# EUROPEAN PATENT SPECIFICATION

(11) **EP 2 292 777 B1**
(45) Date of publication and mention of the grant of the patent: **13.04.2016**
(21) Application number: 10251479.1
(22) Date of filing: 23.08.2010
(51) Int. Cl.: C12N 15/82, C12N 9/10, C12N 15/113

(54) **Transgenic sweet sorghum with altered lignin composition and process of preparation thereof**
Transgene Zuckerhirse mit veränderter Ligninzusammensetzung und Herstellungsverfahren dafür
Sorgho à sucre transgénique avec composition en lignine altérée et son procédé de préparation

(30) Priority: 21.08.2009 US 545699; 12.03.2010 IN CH06652010
(43) Date of publication of application: 09.03.2011
(73) Proprietor: Nagarjuna Energy Private Limited, 500 082 Hyderabad (IN)
(72) Inventor: Basu, Asitava, 721 302 Kharagpur (IN); Maiti, Mrinal Kumar, 721 302 Kharagpur (IN); Kar, Satarupa, 721 302 Kharagpur (IN); Sen, Soumitra Kumar, 721 302 Kharagpur (IN); Pandey, Banibrata, 500 082 Hyderabad (IN)
(74) Representative: Wright, Simon Mark

(56) References cited:
- WO-A2-2008/064289
- WO-A2-2008/102241
- US-B1- 6 441 272

### SEQUENCE LISTING

<110> NAGARJUNA ENERGY PRIVATE LIMITED
<120> TRANSGENIC SWEET SORGHUM WITH ALTERED LIGNIN COMPOSITION AND PROCESS OF PREPARATION THEREOF
<130> N.111831
<150> US 12/545,699
   <151> 2009-08-21
<150> IN 00665/CHE2010
   <151> 2010-03-12
<160> 30
<170> PatentIn version 3.5
<210> 1
   <211> 759
   <212> DNA
   <213> Sorghum bicolor
<400> 1
   atggccgaaa acggcgaaga gcagcaggcg aacggcaacg gcgagcagaa gacccggcat 60
   caggaagtag ggcacaagag cctgctcaag agcgacgagc tctaccagta catcctggac 120
   acgagcgtgt acccgcggga gccggagagc atgaaggagc tccgcgagat caccgccaag 180
   cacccatgga acctgatgac gacctccgcc gacgaggggc agttcctcaa catgctcatc 240
   aagctcatcg gcgccaagaa gaccatggag atccgcgtct acaccggcta ctccctcctt 300
   gctactgcca tggctcttcc cgatgatggc aagattctag ctatggatat taaccgggaa 360
   aactacgaga ttggtcttcc agtgattgaa aaggctggac tggcccacaa gatcgacttc 420
   cgcgagggcc ccgcgctccc cgtcctcgac gacctcatcg ccgacgagaa gaaccacggg 480
   tcgttcgact tcgtcttcgt ggacgccgac aaggacaact acctcaacta ccacgaccgg 540
   ctgctcaagc tggtgaagct ggggggcctc atcggctatg acaacacact gtggaacggg 600
   agcgtcgtgc tgcccgacga cgccccgatg cggaagtaca ttcgcttcta ccgcgatttc 660
   gtcctcgtcc tgaacaaggc gctcgcggcg gatgatcgcg tcgagatctg ccagctcccc 720
   gtcggtgacg gtgtgacgct gtgccggcgc gtcaagtga 759
<210> 2
   <211> 252
   <212> PRT
   <213> Sorghum bicolor
<400> 2
   Met Ala Glu Asn Gly Glu Glu Gln Gln Ala Asn Gly Asn Gly Glu Gln
   1               5                   10                  15
   Lys Thr Arg His Gln Glu Val Gly His Lys Ser Leu Leu Lys Ser Asp
               20                  25                  30
   Glu Leu Tyr Gln Tyr Ile Leu Asp Thr Ser Val Tyr Pro Arg Glu Pro
           35                  40                  45
   Glu Ser Met Lys Glu Leu Arg Glu Ile Thr Ala Lys His Pro Trp Asn
       50                  55                  60
   Leu Met Thr Thr Ser Ala Asp Glu Gly Gln Phe Leu Asn Met Leu Ile
   65                  70                  75                  80
   Lys Leu Ile Gly Ala Lys Lys Thr Met Glu Ile Arg Val Tyr Thr Gly
                   85                  90                  95
   Tyr Ser Leu Leu Ala Thr Ala Met Ala Leu Pro Asp Asp Gly Lys Ile
               100                 105                 110
   Leu Ala Met Asp Ile Asn Arg Glu Asn Tyr Glu Ile Gly Leu Pro Val
           115                 120                 125
   Ile Glu Lys Ala Gly Leu Ala His Lys Ile Asp Phe Arg Glu Gly Pro
       130                 135                 140
   Ala Leu Pro Val Leu Asp Asp Leu Ile Ala Asp Glu Lys Asn His Gly
   145                 150                 155                 160
   Ser Phe Asp Phe Val Phe Val Asp Ala Asp Lys Asp Asn Tyr Leu Asn
                   165                 170                 175
   Tyr His Asp Arg Leu Leu Lys Leu Val Lys Leu Gly Gly Leu Ile Gly
               180                 185                 190
   Tyr Asp Asn Thr Leu Trp Asn Gly Ser Val Val Leu Pro Asp Asp Ala
           195                 200                 205
   Pro Met Arg Lys Tyr Ile Arg Phe Tyr Arg Asp Phe Val Leu Val Leu
       210                 215                 220
   Asn Lys Ala Leu Ala Ala Asp Asp Arg Val Glu Ile Cys Gln Leu Pro
   225                 230                 235                 240
   Val Gly Asp Gly Val Thr Leu Cys Arg Arg Val Lys
   245 250
<210> 3
   <211> 31
   <212> DNA
   <213> Artificial Sequence
<220>
   <223> forward degenerate primer
<220>
   <221> misc_feature
   <222> (23)..(23)
   <223> n is a, c, g, or t
<220>
   <221> misc_feature
   <222> (26)..(26)
   <223> n is a, c, g, or t
<400> 3
   gaattcggat ccaycargar gtnggncaya a 31
<210> 4
   <211> 27
   <212> DNA
   <213> Artificial Sequence
<220>
   <223> reverse degenerate primer
<220>
   <221> misc_feature
   <222> (13)..(13)
   <223> n is a, c, g, or t
<220>
   <221> misc_feature
   <222> (16)..(16)
   <223> n is a, c, g, or t
<400> 4
   gaattcrttc canagngtrt trtcrta 27
<210> 5
   <211> 32
   <212> DNA
   <213> Artificial Sequence
<220>
   <223> forward specific primer
<400> 5
   gaattcggat cccatcagga agtagggcac aa 32
<210> 6
   <211> 29
   <212> DNA
   <213> Artificial Sequence
<220>
   <223> reverse specific primer
<400> 6
   ccgagctcgt tccacagtgt gttgtcata 29
<210> 7
   <211> 54
   <212> PRT
   <213> Zea mays
<400> 7
   Met Ala Thr Thr Ala Thr Glu Ala Ala Pro Ala Gln Glu Gln Gln Ala
   1               5                   10                  15
   Asn Gly Asn Gly Glu Gln Lys Thr Arg His Ser Glu Val Gly His Lys
               20                  25                  30
   Ser Leu Leu Lys Ser Asp Asp Leu Tyr Gln Tyr Ile Leu Asp Thr Ser
           35                  40                  45
   Val Tyr Pro Arg Glu Pro
       50
<210> 8
   <211> 48
   <212> PRT
   <213> Sorghum bicolor
<400> 8
   Met Ala Glu Asn Gly Glu Glu Gln Gln Ala Asn Gly Asn Gly Glu Gln
   1               5                   10                  15
   Lys Thr Arg His Gln Glu Val Gly His Lys Ser Leu Leu Lys Ser Asp
               20                  25                  30
   Glu Leu Tyr Gln Tyr Ile Leu Asp Thr Ser Val Tyr Pro Arg Glu Pro
           35                  40                  45
<210> 9
   <211> 60
   <212> PRT
   <213> Zea mays
<400> 9
   Met Ala Thr Thr Ala Thr Glu Ala Thr Lys Thr Thr Ala Pro Ala Gln
   1 5 10 15
   Glu Gln Gln Ala Asn Gly Asn Gly Asn Gly Glu Gln Lys Thr Arg His
   20 25 30
   Ser Glu Val Gly His Lys Ser Leu Leu Lys Ser Asp Asp Leu Tyr Gln
   35 40 45
   Tyr Ile Leu Asp Thr Ser Val Tyr Pro Arg Glu Pro
   50 55 60
<210> 10
   <211> 56
   <212> PRT
   <213> Oryza sativa
<400> 10
   Met Ala Glu Ala Ala Ser Ala Ala Ala Ala Ala Thr Thr Glu Gln Ala
   1 5 10 15
   Asn Gly Ser Ser Gly Gly Glu Gln Lys Thr Arg His Ser Glu Val Gly
   20 25 30
   His Lys Ser Leu Leu Lys Ser Asp Asp Leu Tyr Gln Tyr Ile Leu Glu
   35 40 45
   Thr Ser Val Tyr Pro Arg Glu His
   50 55
<210> 11
   <211> 60
   <212> PRT
   <213> Zea mays
<400> 11
   Glu Ser Met Lys Glu Leu Arg Glu Val Thr Ala Lys His Pro Trp Asn
   1 5 10 15
   Leu Met Thr Thr Ser Ala Asp Glu Gly Gln Phe Leu Asn Met Leu Ile
   20 25 30
   Lys Leu Ile Gly Ala Lys Lys Thr Met Glu Ile Gly Val Tyr Thr Gly
   35 40 45
   Tyr Ser Leu Leu Ala Thr Ala Leu Ala Leu Pro Glu
   50 55 60
<210> 12
   <211> 60
   <212> PRT
   <213> Sorghum bicolor
<400> 12
   Glu Ser Met Lys Glu Leu Arg Glu Ile Thr Ala Lys His Pro Trp Asn
   1               5                   10                  15
   Leu Met Thr Thr Ser Ala Asp Glu Gly Gln Phe Leu Asn Met Leu Ile
               20                  25                  30
   Lys Leu Ile Gly Ala Lys Lys Thr Met Glu Ile Arg Val Tyr Thr Gly
           35                  40                  45
   Tyr Ser Leu Leu Ala Thr Ala Met Ala Leu Pro Asp
       50                  55                  60
<210> 13
   <211> 60
   <212> PRT
   <213> Zea mays
<400> 13
   Glu Ser Met Lys Glu Leu Arg Glu Ile Thr Ala Lys His Pro Trp Asn
   1               5                   10                  15
   Leu Met Thr Thr Ser Ala Asp Glu Gly Gln Phe Leu Asn Met Leu Ile
               20                  25                  30
   Lys Leu Ile Gly Ala Lys Lys Thr Met Glu Ile Gly Val Tyr Thr Gly
           35                  40                  45
   Tyr Ser Leu Leu Ala Thr Ala Leu Ala Leu Pro Glu
       50                  55                  60
<210> 14
   <211> 60
   <212> PRT
   <213> Oryza sativa
<400> 14
   Glu Cys Met Lys Glu Leu Arg Glu Val Thr Ala Asn His Pro Trp Asn
   1               5                   10                  15
   Leu Met Thr Thr Ser Ala Asp Glu Gly Gln Phe Leu Asn Leu Leu Leu
               20                  25                  30
   Lys Leu Ile Gly Ala Lys Lys Thr Met Glu Ile Gly Val Tyr Thr Gly
           35                  40                  45
   Tyr Ser Leu Leu Ala Thr Ala Leu Ala Ile Pro Asp
       50                  55                  60
<210> 15
   <211> 60
   <212> PRT
   <213> Zea mays
<400> 15
   Asp Gly Thr Ile Leu Ala Met Asp Ile Asn Arg Glu Asn Tyr Glu Leu
   1               5                   10                  15
   Gly Leu Pro Cys Ile Glu Lys Ala Gly Val Ala His Lys Ile Asp Phe
               20                  25                  30
   Arg Glu Gly Pro Ala Leu Pro Val Leu Asp Asp Leu Ile Ala Glu Glu
           35                  40                  45
   Lys Asn His Gly Ser Phe Asp Phe Val Phe Val Asp
       50                  55                  60
<210> 16
   <211> 60
   <212> PRT
   <213> Sorghum bicolor
<400> 16
   Asp Gly Lys Ile Leu Ala Met Asp Ile Asn Arg Glu Asn Tyr Glu Ile
   1               5                   10                  15
   Gly Leu Pro Val Ile Glu Lys Ala Gly Leu Ala His Lys Ile Asp Phe
               20                  25                  30
   Arg Glu Gly Pro Ala Leu Pro Val Leu Asp Asp Leu Ile Ala Asp Glu
           35                  40                  45
   Lys Asn His Gly Ser Phe Asp Phe Val Phe Val Asp
       50                  55                  60
<210> 17
   <211> 60
   <212> PRT
   <213> Zea mays
<400> 17
   Asp Gly Thr Ile Leu Ala Met Asp Ile Asn Arg Glu Asn Tyr Glu Leu
   1               5                   10                  15
   Gly Leu Pro Cys Ile Asn Lys Ala Gly Val Gly His Lys Ile Asp Phe
               20                  25                  30
   Arg Glu Gly Pro Ala Leu Pro Val Leu Asp Asp Leu Val Ala Asp Lys
           35                  40                  45
   Glu Gln His Gly Ser Phe Asp Phe Ala Phe Val Asp
       50                  55                  60
<210> 18
   <211> 60
   <212> PRT
   <213> Oryza sativa
<400> 18
   Asp Gly Thr Ile Leu Ala Met Asp Ile Asn Arg Glu Asn Tyr Glu Leu
   1               5                   10                  15
   Gly Leu Pro Ser Ile Glu Lys Ala Gly Val Ala His Lys Ile Asp Phe
               20                  25                  30
   Arg Glu Gly Pro Ala Leu Pro Val Leu Asp Gln Leu Val Glu Glu Glu
           35                  40                  45
   Gly Asn His Gly Ser Phe Asp Phe Val Phe Val Asp
       50                  55                  60
<210> 19
   <211> 60
   <212> PRT
   <213> Zea mays
<400> 19
   Ala Asp Lys Asp Asn Tyr Leu Asn Tyr His Glu Arg Leu Leu Lys Leu
   1               5                   10                  15
   Val Lys Leu Gly Gly Leu Ile Gly Tyr Asp Asn Thr Leu Trp Asn Gly
               20                  25                  30
   Ser Val Val Leu Pro Asp Asp Ala Pro Met Arg Lys Tyr Ile Arg Phe
           35                  40                  45
   Tyr Arg Asp Phe Val Leu Val Leu Asn Lys Ala Leu
       50                  55                  60
<210> 20
   <211> 60
   <212> PRT
   <213> Sorghum bicolor
<400> 20
   Ala Asp Lys Asp Asn Tyr Leu Asn Tyr His Asp Arg Leu Leu Lys Leu
   1               5                   10                  15
   Val Lys Leu Gly Gly Leu Ile Gly Tyr Asp Asn Thr Leu Trp Asn Gly
               20                  25                  30
   Ser Val Val Leu Pro Asp Asp Ala Pro Met Arg Lys Tyr Ile Arg Phe
           35                  40                  45
   Tyr Arg Asp Phe Val Leu Val Leu Asn Lys Ala Leu
       50                  55                  60
<210> 21
   <211> 60
   <212> PRT
   <213> Zea mays
<400> 21
   Ala Asp Lys Asp Asn Tyr Leu Asn Tyr His Glu Arg Leu Leu Lys Leu
   1               5                   10                  15
   Val Arg Pro Gly Gly Leu Ile Gly Tyr Asp Asn Thr Leu Trp Asn Gly
               20                  25                  30
   Ser Val Val Leu Pro Asp Asp Ala Pro Met Arg Lys Tyr Ile Arg Phe
           35                  40                  45
   Tyr Arg Asp Phe Val Leu Ala Leu Asn Ser Ala Leu
       50                  55                  60
<210> 22
   <211> 60
   <212> PRT
   <213> Oryza sativa
<400> 22
   Ala Asp Lys Asp Asn Tyr Leu Asn Tyr His Glu Arg Leu Met Lys Leu
   1               5                   10                  15
   Val Lys Val Gly Gly Leu Val Gly Tyr Asp Asn Thr Leu Trp Asn Gly
               20                  25                  30
   Ser Val Val Leu Pro Ala Asp Ala Pro Met Arg Lys Tyr Ile Arg Tyr
           35                  40                  45
   Tyr Arg Asp Phe Val Leu Glu Leu Asn Lys Ala Leu
       50                  55                  60
<210> 23
   <211> 24
   <212> PRT
   <213> Zea mays
<400> 23
   Ala Ala Asp Asp Arg Val Glu Ile Cys Gln Leu Pro Val Gly Asp Gly
   1               5                   10                  15
   Val Thr Leu Cys Arg Arg Val Lys
               20
<210> 24
   <211> 24
   <212> PRT
   <213> Sorghum bicolor
<400> 24
   Ala Ala Asp Asp Arg Val Glu Ile Cys Gln Leu Pro Val Gly Asp Gly
   1               5                   10                  15
   Val Thr Leu Cys Arg Arg Val Lys
               20
<210> 25
   <211> 24
   <212> PRT
   <213> Zea mays
<400> 25
   Ala Ala Asp Asp Arg Val Glu Ile Cys Gln Leu Pro Val Gly Asp Gly
   1               5                   10                  15
   Val Thr Leu Cys Arg Arg Val Lys
               20
<210> 26
   <211> 24
   <212> PRT
   <213> Oryza sativa
<400> 26
   Ala Ala Asp His Arg Val Glu Ile Cys Gln Leu Pro Val Gly Asp Gly
   1               5                   10                  15
   Ile Thr Leu Cys Arg Arg Val Lys
               20
<210> 27
   <211> 6
   <212> DNA
   <213> Artificial Sequence
<220>
   <223> TATA box consensus sequence
<400> 27
   tataat 6
<210> 28
   <211> 6
   <212> DNA
   <213> Artificial Sequence
<220>
   <223> GC box consensus sequence
<400> 28
   gggcgg 6
<210> 29
   <211> 30
   <212> DNA
   <213> Artificial Sequence
<220>
   <223> gene specific forward primer
<400> 29
   atgaaaaagc ctgaactcac cgcgacgtct 30
<210> 30
   <211> 30
   <212> DNA
   <213> Artificial Sequence
<220>
   <223> gene specific reverse primer
<400> 30
   gcatcagctc atcgagagcc tgcgcgacgg 30

## Description

### Field of the Invention:

The present invention is in the field of biotechnology and more particularly development of a plant having altered lignin composition.

### Background and prior art:

Sweet sorghum, *Sorghum bicolor* (L.) Moench is the only crop that provides grain and stem that can be used for the production of alcohol, sugar, syrup, fuel etc. Sweet sorghum offers following advantages over the other crops:
   - Growing period (about 4 months) and water requirement (8000 m³ over two crops) are 4 times lower than those of sugarcane (12 to 16 months and 36000 m³ respectively).
   - Cost of cultivation of sweet sorghum is 3 times lower than sugarcane.
   - Seed propagated.
   - Suitable for mechanized crop production.
   - The ethanol production process from sweet sorghum is eco-friendly compared to that from molasses.
   - Ethanol burning quality is superior - less sulphur than from sugarcane and high octane rating.

*See* International Crops Research Institute for the Semi-Arid Tropics (ICRISAT) brochure, "Sweet Sorghum: Food, Feed, Fodder and Fuel Crop," published 2006.

But the primary concern for using this plant is the presence of lignin, which adversely affects the process of extraction of beneficial materials. Beside the sugary juice, the stem offer great potential for biofuel production. However, due to the presence of lignin, as in other crop plants, affect the separation of the available sugars such as xylose, arabinose, glucose etc.

Lignin is a complex phenylpropanoid polymer. Plants comprise about 25-30% lignin based on the dry weight. Lignin is primarily deposited in the cell walls of supporting and conductive tissues, such as fibers and tracheary elements. The mechanical rigidity of lignin strengthens these tissues so that the tracheary elements can endure the negative pressure generated from transpiration without collapse of the tissue.

However, lignin is unfavorable in various other aspects. Lignin decreases the digestibility of animal forage crops and must be removed during pulping and paper making, which requires the use of chemicals hazardous to the environment. Lignin also appears to have a negative impact on the utilization of plant and tree biomass.

Lignin is considered to be dehydrogenatively polymerized from the monolignols p-coumaryl alcohol, coniferyl alcohol, and sinapyl alcohol. These monolignols are synthesized through the phenylpropanoid pathway. Structurally these monolignols differ only by the methoxy group at the 3C and 5C positions of the aromatic ring. Varying proportion of the monolignols determine the type of lignin such as H, G, S lignin. G lignin offers more resistance than S during enzymatic saccharification. G lignins are more condensed due to more numbers of intermolecular linkages, thereby showing more resistance.

Therefore, it is desirable to develop a sweet sorghum plant with altered lignin composition, said composition comprising more of S and less G lignin as compared to the wild. In order to achieve that it is also required that the modified plant must have more biomass content and its bio-chemical architecture is favorable for downstream processing.

Several approaches have been taken to decrease or alter the composition of lignin content to increase S/G ratio. However, the results have found to be contradictory, possibly due to lack of understanding of lignin biosynthetic pathway and due to inappropriate suitable approaches for down regulation of the lignin biosynthetic enzyme activity including choice of transgene, promoter used, construction of cassettes and above all, selection of transformants. Regulation of early steps enzymes like phenylalanine ammonia lyase, cinnamate 4-hydroxylase, 4-hydroxycinnamate CoA ligase reduced lignin content. However, it leads to pleiotropic effects including altered leaf shape, localized fluorescent lesion, stunted growth, reduced pollen activity, altered flower morphology and pigmentation, reduced level of soluble phenylpropanoids, decrease in S/G ratio etc (Elkind et al, 1990; Bate et al, 1994; Sewalt et al, 1997). Similar effects by other workers to alter or modify the S/G ratio have resulted in phenotypically defective plants. It was demonstrated that down regulation of caffeic acid O-methyltransferase activity could result dramatic decrease in syringyl lignin biosynthesis but with little effect on the synthesis of guaiacyl lignin, which is undesirable as the latter are more resistant to chemical degradation. However, till date any alteration in lignin composition in sweet sorghum is carried out primarily by down regulating COMT gene.

It is therefore of interest to develop a modified sweet sorghum plant having altered lignin composition and biochemically suitable for downstream processing. In order to achieve the same, the inventor had attempted to find out genes capable of altering lignin composition in sweet sorghum plant and said plant is susceptible to easy enzymatic degradation compared to control plant as well as COMT down-regulated sorghum plants generated in the present study through similar gene silencing approach.

### Summary of the invention:

The present invention provides a process for producing a transgenic *Sorghum bicolor* plant having increased biomass comprising:
i) transforming or transfecting a *Sorghum bicolor* cell with a double strand mediated gene silencing cassette comprising a nucleic acid represented by SEQ ID NO. 1, wherein said nucleic acid is operably associated with a promoter, and wherein the nucleic acid is in the sense-antisense orientation to generate RNAi in vivo to at least a portion of SEQ ID NO. 1; and
ii) growing a *Sorghum bicolor* plant from said cell.

The present invention also provides use of the plant described above comprising the step of harvesting the grain and/or stems of the plant or use of the plant described above in the production of alcohol, sugar, syrup or biofuel.

Disclosed herein is a polypeptide sequence capable of altering the lignin composition in sweet sorghum. Down regulation of said polynucleotide in sweet sorghum plants causes formation of more S-lignin as compared to G-lignin, higher free sugar content and shows better morphological characteristics in modified plants as compared to the wild plant. Computer-based analysis of the isolated sequence reveals a similarity with CCoAOMT of arthologous plants. However, to date no literature discloses the presence of CCoAOMT and its role in modulation of lignin composition in sweet sorghum. Hereinafter the polynucleotide sequence is referred to as SEQ ID NO 1, which has CCoAOMT-like properties, whose sequence has been partially identified.

Disclosed herein is an isolated polynucleotide sequence, where the sequence is obtained from *Sorghum bicolor* encoding Caffeoyl-CoA-O-methyltransferase (CCoAOMT), and is represented by SEQ ID NO 1. According to some aspects, the DNA may be cDNA.

The invention relates to a process comprising the use of a RNAi mediated gene silencing construct comprising a suitable fragment of nucleic acid represented by SEQ ID NO 1, where the nucleic acid is operably associated with a promoter, wherein the nucleic acid fragment is in the double stranded sense-antisense orientation and facilitate *in vivo* production of RNAi to at least a portion of a SEQ ID NO 1. Vectors comprising the construct are disclosed herein, and in some aspects the vector may be a pUC18 vector.

Also disclosed herein is a transgenic plant, seed, grain or stems comprising a construct described above.

A further aspect relates to forward and reverse primers represented by SEQUENCE ID NO 3 and 4 or 5 and 6. The primers are useful in amplifying CCoAOMT.

Surprisingly, it has been found that down regulation of SEQ ID NO 1 in sweet sorghum results in a plant that is superior to the plants obtained by down regulating COMT in respect of the biomass, starch, and free sugar content and phenotypical properties.

### Brief description of Figures:

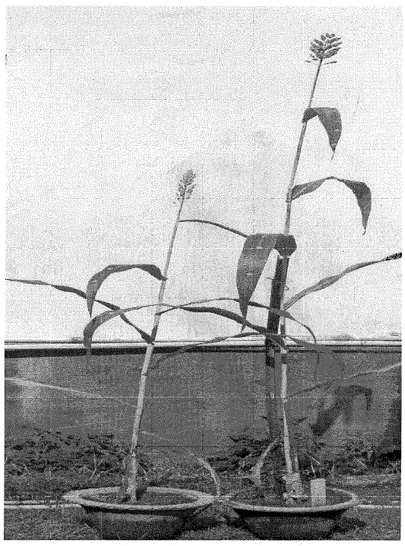

- Fig. 1:: Enhanced growth of the anti-CCoAOMT-like transformed sorghum T0 plant (right) in comparison to control plant (left).

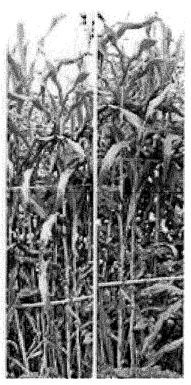

- Fig. 2:: Enhanced growth of transgenic sorghum (T1 plant) with down regulated CCoAOMT-like gene represented by SEQ ID NO 1 in field.

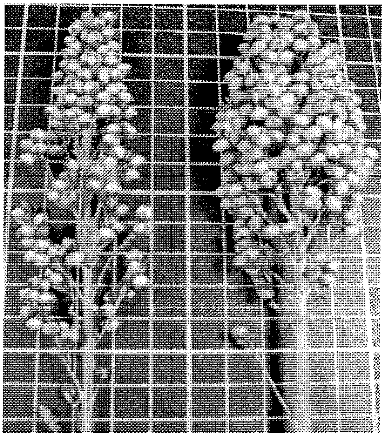

- Fig. 3:: Panicle of control plant (left) and CCoAOMT-like gene transformed plant (right).

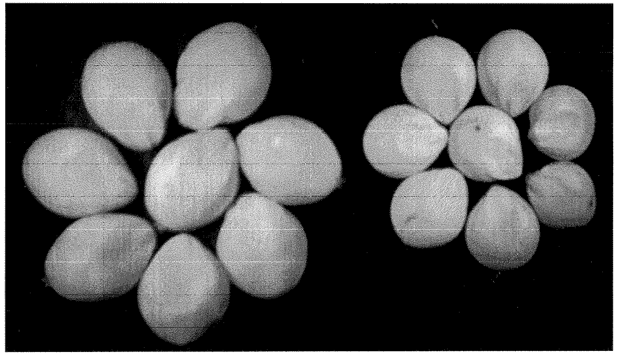

- Fig. 4:: Variation in seed size in anti-CCoAOMT plants (left) in comparison to control plants (right)

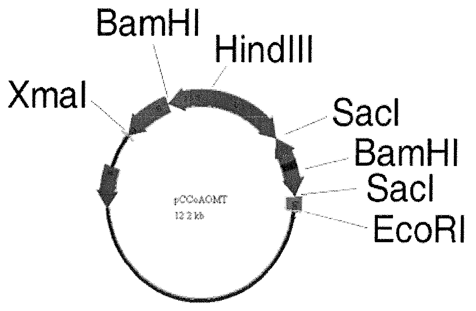

- Fig. 5:: Double strand mediated anti-CCoAOMT construct that will facilitate the specific *in vivo* production of RNAi against endogenous CCoAOMT gene. Hindlll/Sacl: Ubiquitin promoter (U); Sacl/Sacl: double strand mediated anti-CCoAOMT construct; Sacl/EcoRI: Nopaline synthase terminator (T); Hindlll/BamHI: 2x 35S promoter; BamHI/Xmal: hptII gene (H) for plant selection and K: nptII gene for bacterial selection.
- Fig. 6:: Selection of plants by PCR amplification of marker genes of transformed lines of COMT and CCoAOMT.
- Fig. 7:: Southern analysis of T₁ progenies of CCoAOMT along with the respective To transformants and a control plant showing inheritance of the integrated transgenes.

### Detailed description of the present invention:

Accordingly, disclosed herein is a sweet sorghum plant characterized by altered lignin composition compared to a wild plant and this is achieved by down regulating the expression of polynucleotide represented by SEQ ID NO 1 in sweet sorghum.

One of the aspects is RNAi mediated gene silencing of the endogenous gene represented by SEQ ID NO 1 such that when the exogenous nucleic acid is transcribed, the activity of the endogenous SEQ ID NO 1 is down regulated.

Also disclosed herein is a process of producing a modified sweet sorghum plant having altered lignin composition. Said process comprises transfecting a plant cell with double strand RNA (dsRNA) mediated gene silencing cassettes using nucleotide sequence represented in SEQ ID NO 1 that facilitates the *in vivo* production of RNAi against the endogenous gene and associated with reduced content of G-lignin as compared to the wild plant.

Two transgenic lines were developed, one with anti-COMT and other with anti-SEQ ID NO 1, and the anti-SEQ ID NO 1 transformed plant showed desirable as well as beneficial phenotypic characters like enhanced growth, panicle shape, increased number of seed per panicle, increased size and weight and thereby provide the more biomass as raw material for downstream use and higher starch and free sugar content as compared to anti-COMT transformed plant. The plants transformed with anti-COMT and anti-SEQ ID NO 1 construct hereinafter referred to as "C-plant" and "T-plant" respectively.

The genetic modification of sweet sorghum with SEQ ID NO 1 has a far reaching implication. The intended use of sorghum biomass would be in fermentation process. In fermentation process, it is very important to have biomass with high sugar content and preferably soluble sugar. It is pertinent to mention here that upon morphological and biochemical analysis of the modified sorghum plant, a substantial increase in the biomass and cellulose content was observed.

### Quest for a new gene in sweet sorghum capable of altering lignin composition:

### A] Isolation of total RNA from mature Sorghum stem by hot phenol method:

1gm fresh sorghum stem was taken and chopped into small pieces and crushed in mortar with pestle in presence of liquid nitrogen still it becomes powdery. A pinch of poly vinyl pyrrolidone (PVP) and 200µl β-marcapto ethanol (β-ME) were added with the powder. 5ml RNA extraction buffer and 5ml saturated phenol mixed previously and heated in 80°C water bath was added to the powder, mixed well and allowed to thaw. Thawed sample then transferred in a centrifuge tube and heated in 80ºC water bath for 20 minutes. The tube was kept in room temperature for 5-10 minutes and 5ml of chloroform was added to it and the tube was vortexed very well. Then the tube was centrifuged at 10,000 rpm for 10 minutes at room temperature, supernatant was taken in a corex tube. 1/10^{th} volume of Na-acetate and 2 volume of chilled ethanol were added to it and allowed to precipitate at - 20°C overnight. Next day the corex tube was centrifuged at 10,000 rpm for 10 minutes at 4ºC. Supernatant was discarded and the pellet was air dried. Pellet was dissolved in 1ml DEPC treated water and distributed into two microfuge tubes. Equal volume of saturated phenol added, shaked gently and centrifuged at 10,000 rpm for 5 minutes and the supernatant was taken. Phenol step was repeated still the supernatant becomes clear. Followed by phenol step equal amount of chloroform was added and centrifuged at 10,000 rpm for 5 minutes, supernatant was taken. 1/10^{th} volume of Na-acetate and 2 volume of chilled ethanol were added to it and allowed to precipitate at -20ºC for 1 hour. It was then centrifuged at 10,000 rpm for 10 minutes and supernatant was discarded, the pellet was washed with 70% ethanol, dried well and finally dissolved in formamide and kept in -70ºC for preservation.

### Composition of RNA extraction buffer (pH 8):

| | |
|---|---|
| LiCl | - 100 mM |
| TRIS | - 100 mM |
| EDTA | - 10 mM |
| SDS | - 1 % |

### Purification of mRNA by oligo (dT) cellulose matrix:

100mg of oligo (dT) cellulose was suspended in 1ml elution buffer and kept it at room temperature overnight. Elution buffer was removed by short spin. The column was equilibrated with 1ml binding buffer. Binding buffer was removed by short spin. 1mg RNA sample in formamide was precipitataed on the previous night and washed with 70% ethanol, dried and dissolved thoroughly in 1ml binding buffer and heated at 65ºC for 5 minutes, and quickly chilled on ice for 5 minutes. Then the RNA sample was loaded into oligo dT cellulose matrix. Binding was allowed for 30 minutes with gentle shaking. This suspension was centrifuged by short spin. The column was washed 3 times for 30 minutes each with 1ml wash buffer to remove the binding buffer. Poly (A⁺) mRNA was extracted twice with 200 µl elution buffer, centrifuged at 13,000 rpm for 30 seconds. The eluted pool was readjusted with 0.5M NaCl by adding NaCl accordingly. The whole procedure was repeated twice more from the step of addition of binding buffer to elution. Re-bound, re-washed, re-eluted pooled sample was precipitated with 1/10^{th} volume of Na-acetate and 2.5^{th} volume of ethanol for 1 hour. Pellet was washed with 70% ethanol, dried and resuspended in DEPC treated water.

### Composition of the buffers:

### a) Oligo(dT) binding buffer:

| | |
|---|---|
| TRIS-HCl (pH 7.5) | - 10 mM |
| NaCl | - 500 mM |
| EDTA | - 1 mM |
| SDS | - 0.5 % |

### b) Oligo(dT) wash buffer:

| | |
|---|---|
| TRIS-HCI (pH 7.5) | - 10 mM |
| NaCl | - 100 mM |
| EDTA | - 1 mM |

### c) Oligo(dT) elution buffer:

| | |
|---|---|
| TRIS-HCl (pH 7.5) | - 10 mM |
| EDTA | - 1 mM |

### Synthesis of cDNA from mRNA isolated from mature stem tissue:

Using reverse transcription, cDNA was prepared from mRNA, which was isolated from sweet sorghum. A primer is annealed to the mRNA providing a free 3' end that can be used for extension by the enzyme reverse transcriptase. The enzyme engages in the usual 5' to 3' elongation, as directed by complementary base pairing with the mRNA template to form a hybrid molecule, consisting of a template RNA strand base paired with the complementary cDNA strand. After degradation of the original mRNA, a DNA polymerase was used to synthesize the complementary DNA strand to convert the single stranded cDNA into a duplex cDNA.

Desired complete cDNA was isolated using PCR (Polymerase Chain reaction) with degenerated primers designed from conserved amino acid sequence of the gene from heterologous plant system followed by 5' and 3' RACE (Rapid Amplification of cDNA Ends). cDNA was synthesized with anchored oligo(dT)₁₈ primer and random hexamer primer using Standard RT-PCR Reaction kit by Roche. In a sterile, nuclease free, thin walled PCR tube on ice, template primer mixture was prepared for one 20µl reaction by adding the components in the order listed below.

### Template-primer mix:

| **Component** | **Volume** | **Final conc.** |
|---|---|---|
| Poly(A)⁺ mRNA | 1 µl | 10 ng poly(A)⁺ mRNA |
| Anchored-oligo(dT)₁₈ primer, 50 pmol/µl | 1 µl | 2.5 µM |
| Random hexamer primer, 600 pmol/µl | 2 µl | 60 µM |
| PCR grade water | 9 µl | |
| **Total volume** | **13 µl** | |

The template-primer mixture was heated for 10 minutes at 65ºC in a thermal block cycler with a heated lid to denature the secondary structures. Immediately the tube was placed on ice. The remaining components of the RT mix were added in the order listed below.

| **Component** | **Volume** | **Final conc.** |
|---|---|---|
| Transcriptor Reverse Transcriptase Reaction Buffer (5X) | 4 µl | 1X (8 mM MgCl₂) |
| Protector RNase Inhibitor, 40 U/µl | 0.5 µl | 20 U |
| Deoxynucleotide Mix, 10 mM each | 2 µl | 1 mM each |
| Transcriptor Reverse Transcriptase, 20 U/µl | 0.5 µl | 10 U |
| **Final volume** | **20 µl** | |

The reagents were mixed carefully and centrifuged briefly and kept at thermal block cycler for 10 minutes at 25ºC, followed by 30 minutes at 55ºC. Transcriptor Reverse Transcriptase was inactivated by heating it to 85ºC for 5 minutes. The reaction was stopped by placing the tube on ice.

### Design and synthesis of primers and PCR amplification:

Two degenerated 5' and 3' primers were designed. A BamHl site in the 5'-primer and an EcoRl site in the 3'-primer were introduced for cloning of the PCR amplified fragment. Translated amino acid sequence analysis of the amplified fragment showed identity with the heterologous CCoAOMT. The sequences of the degenerate primers are:
Forward primer: 5' gaa ttc gga tcc a(c/t)c a(a/g)g a(a/g)g tng gnc a(c/t)a a 3' (SEQ ID NO 3)
Reverse primer: 5' gaa ttc (g/a)tt cca nag ngt (g/a)tt (g/a)tc (g/a)ta 3' (SEQ ID NO 4)

| **Steps** | **Total cycle** | **Temperature(ºC)** | **Duration(min)** |
|---|---|---|---|
| Primary denaturation | 1 | 94 | 4 |
| Denaturation | | 94 | 0.4 |
| Annealing | 30 | 58 | 0.4 |
| Extension | | 72 | 1 |
| Final extension | 1 | 72 | 7 |

### Cloning of the partial SEQ ID NO 1 fragment:

Both the PCR amplified fragment and vector pUC18 was digested with restriction enzymes BamHI and EcoRI for cloning as it was introduced in the primers. Both the digested vector and amplified fragment were purified by LMP agarose gel. Purified products were subjected to ligation with T4 DNA ligase. A part of the ligation mixture was then transformed in DH10B competent cells and plated on ampicillin (100µg/ml). Transformed colonies were selected. The recombinant plasmid DNA was isolated from the clones and sequenced. The cloned fragment was further amplified with specific primers (SEQ ID NO 5 and 6) and digested with BamHl and Sacl and joined in sense-antisense orientation with the help of arbitrary linker having BamHl and Bglll sites. This sense-antisense fragment was then placed in a binary vector under a suitable promoter to generate dsRNA induced cassette for CCoAOMT for *Agrobacterium* mediated transformation in plant.

### The sequences of the specific primers are:

Forward primer: 5' gaa ttc gga tcc cat cag gaa gta ggg cac aa 3' (SEQ ID NO 5)
Reverse primer: 5' cc gag ctc gtt cca cag tgt gtt gtc ata 3' (SEQ ID NO 6)

### Isolation of full-length SEQ ID NO 1:

The full-length SEQ ID NO 1 was isolated through amplification of 5' and 3' ends from cDNA of sorghum using 2^{nd} generation 5'/3' RACE kit in stepwise manner. The fragments were then cloned and characterized. The primers were generated from the identified nucleotide sequence of partial SEQ ID NO 1.

### Cloning of the SEQ ID NO 1 in TA-vector:

The PCR amplified fragment was purified by LMP agarose gel and cloned in TA vectors. A part of the ligation mixture was then transformed in DH10B competent cells and plated on ampicillin (100µg/ml). Transformed colonies were selected and characterized by sequencing.

### Characterization of the clones and software based analysis of the sequenced clones:

Selected white clones were initially characterized by isolating plasmid DNA and restriction digestion with BamHl and EcoRl for the presence of the insert. Then three of these clones for each fragments were sequenced by Big Dye Terminator method of sequencing for further confirmation using software based analytical system like NCBI blast analysis and Clustal W alignment.

**Nucleotide sequence as obtained from sequencing result:** The sequence of the gene was found to be 759 bases. The coding DNA sequence of the gene was found to start from base at 1 and end at base 759. The nucleotide sequence hereinafter referred to as SEQ ID NO 1 and is as follows:

### Computer based analysis of the sequence for further characterization:

The DNA sequence was then translated to get the amino acid sequence using Jellyfish software. The translated sequence is:

This translated amino acid sequence was then subjected to blast analysis using blastP in NCBI and maximum homology was found with its close relative CCoAOMT of *Zea mays.* Thus, the sequence was further analyzed for homology with the CCoAOMT isoforms of maize available in the Genbank database using Clustal W software (shown below) and revealed 95 % identity with maize CCoAOMT isoform.
Maize CCoAOMT1 AADDRVEICQLPVGDGVTLCRRVK (SEQ ID NO 23)
Sorghum CCoAOMT AADDRVEICQLPVGDGVTLCRRVK (SEQ ID NO 24)
Maize CCoAOMT2 AADDRVEICQLPVGDGVTLCRRVK (SEQ ID NO 25)

### Assessing the role of SEQ ID NO 1 in sweet sorghum:

The sequence was then subjected to NCBl blast for identification of its resemblance with the sequence available in the database and found to be highly identical (97% identity) with a hypothetical protein of sorghum. Though this hypothetical protein has been considered to similar to CCoAOMT gene, but its functionality has not yet been tested. Thus, we have taken an approach to establish the effect of isolated gene, as the nucleotide sequence of a gene is more important rather than its functionality in antisense strategy, on down-regulation of CCoAOMT *in-planta.*

### Preparations of Construct comprising SEQ ID NO 1:

**Promoter:** Transcription of DNA into mRNA is regulated by a region of DNA referred to as the promoter. The promoter region contains sequence of bases that signals RNA polymerase to associate with the DNA, and to initiate the transcription of mRNA using one of the DNA strands as a template to make a corresponding complementary strand of RNA. Since the 5' region of the RNA strand is complementary to the 3' region, it will generate a double-stranded RNA, which subsequently degraded using machinery responsible for the production short interfering RNA (RNAi). Promoter sequence include the TATA box consensus sequence (TATAAT (SEQ ID NO 27)), which is usually 20-30 base pair (bp) upstream (by convention -30 to -20bp relative to the transcription start site) of the transcription start site. The TATA box is the only upstream promoter element that has a relatively fixed location with respect to the start point. The CAAT box consensus sequence is centered at -75, but function at distances that vary considerably from the start point and in either orientation. Another common promoter element is the GC box at -90 which contains consensus sequence GGGCGG (SEQ ID NO 28). It may occur in multiple copies and in either orientation. Other sequence conferring maximum efficiency may also be found in the promoter region. In promoter and structural gene combinations, the promoter is preferably in positioned about the same distance from the heterologous transcription start site as it is from the transcription start site in its natural setting. The promoter can be either constitutive or inducible.

The maize ubiquitin promoter used in the present investigation has been shown to be highly active and constitutively expressed in most tissues. It contains the first intron of the maize ubiquitin gene for selective expression in plants. The promoter was cloned in the vector at Hindlll/BamHl site under which the dsRNA inducing fragment of SEQ ID NO 1 was placed. A selectable marker gene, hygromycin phosphotransferase, under 2x 35S promoter was included to allow selection of plant cells bearing the desired construct.

Generation of dsRNA construct was made by joining the suitable region of the desired cDNA fragment in sense and antisense orientation through linker. This construct is introduced into an expression vector for transformation of sorghum plants to find out what role it plays in the metabolic pathway. The vector preferably contains a prokaryotic origin of replication having a broad host range. A selectable marker should also be included to allow selection of bacterial cells bearing the desired construct. Suitable prokaryotic selectable markers include resistance to antibiotics such as kanamycin.

Other DNA sequences encoding additional functions may also be present in the vector, as is known in the art. For instance, in the case of *Agrobacrerium* transformations, T-DNA sequences will also be included for subsequent transfer to plant chromosomes.

For expression in plant, a binary vector was used in which gene of interest can be introduced. The recombinant expression cassettes will contain in addition to desired sequences, a plant promoter region, a transcription initiation site and a transcription terminator sequence. Unique restriction enzyme site at the 5' and 3' ends of the cassettes are typically included to allow for easy insertion into a pre-existing vector. Sequences controlling eukaryotic gene expression are well known in the art.

### Generation of transgenic plants:

Two transgenic lines were developed. The plants transformed with anti-COMT construct hereinafter referred to as "C-plant" and plant having anti-SEQ ID NO 1 referred to as "T-plant". T-plant showed desirable as well as beneficial phenotypic characters like enhanced growth, panicle shape, increased number of seed per panicle, increased size and weight, and thereby provides more biomass as raw material for downstream use.

### Transformation of sweet sorghum (Sorghum bicolor):

Seeds surface sterilized with Tween 20 (5min) and 0.2% mercuric chloride (7min) were washed with sterile distilled water. Then seeds were incubated on sterile filter paper soaked with sterile distilled water in Petri plates. After 3 days incubation in dark the shoot tips generated were excised and infected with infection medium having *Agrobacterium* suspension in it for 20 min. The explants were inoculated on co-cultivation medium and kept in dark for 3 days at 25°C. The explants were occasionally washed with cefotaxime and distilled water to prevent bacterial contamination and transferred on MS with 2mg/l 2,4-D, 30gm/l sucrose and 250mg/l cefotaxime and kept in dark for 12 days for callus formation.

Callus portion at the cut ends of shoot tips were excised and transferred to regeneration medium with hygromycin selection (MS with 30g/l sucrose, 2mg/l BAP and 2mg/l hygromycin) and kept them in 2:1 light/dark periodic condition at 28°C. After 2 weeks, green calli were transferred on the same medium containing higher concentration of selection marker (4mg/l hygromycin). Shoots obtained were transferred on the same medium with higher concentration of hygromycin (5mg/l) for 2 months with periodic subculturing every 2 weeks. Elongated shoots were allowed to rooting medium for root generation. Full grown plantlets were finally selected on ½ MS liquid medium with 6mg/l hygromycin. Plantlets generated from a single callus were described as single line.

### The Agrobacterium-mediated transformation:

The Agrobacterium-mediated transformation process of the invention can be broken into several steps. The basic steps include an infection step; a co-cultivation step; an optional resting step; a selection step; and a regeneration step.

In the infection step, the cells to be transformed are isolated and exposed to Agrobacterium. If the target cells are immature embryos, the embryos are isolated and the cells contacted with a suspension of *Agrobacterium.* As noted above, the *Agrobacterium* has been modified to contain a gene or nucleic acid of interest. The nucleic acid is inserted into the T-DNA region of the vector. General molecular techniques used in the invention are provided, for example, by Sambrook et al. (eds.) Molecular Cloning: A Laboratory Manual, 1989, Cold Spring Harbor Laboratory Press, Cold Spring Harbor, N.Y

The concentration of *Agrobacterium* used in the infection step and co-cultivation step can affect the transformation frequency. Likewise, very high concentrations of Agrobacterium may damage the tissue to be transformed, such as the immature embryos, and result in a reduced callus response. Thus, the concentration of *Agrobacterium* useful in the methods of the invention may vary depending on the *Agrobacterium* strain utilized, the tissue being transformed, the sorghum genotype being transformed, and the like. To optimize the transformation protocol for a particular sorghum line or tissue, the tissue to be transformed, (immature embryos, for example), can be incubated with various concentrations of *Agrobacterium.* Likewise, the level of marker gene expression and the transformation efficiency can be assessed for various *Agrobacterium* concentrations. While the concentration of *Agrobacterium* may vary, generally optical density 0.7 to 1.0 at 600nm was used in the present invention.

The tissue to be transformed is generally added to the *Agrobacterium* suspension in a liquid contact phase containing a concentration of Agrobacterium to optimize transformation efficiencies. The contact phase facilitates maximum contact of the cells/tissue to be transformed with the suspension of *Agrobacterium.* The cells are contacted with the suspension of *Agrobacterium* for a period of at least about three 3 minutes to about 15 minutes, preferably about 4 minutes to about 10 minutes, more preferably about 5 minutes to about 8 minutes

The liquid contact phase of the infection step takes place in a liquid solution MS media along with 68.5 g/l sucrose, 36 g/l glucose, 100 µM acetosyringone and the pH adjusted to 5.2. The other media used in this invention are: Co-cultivation media (MS with 20g/l sucrose, 10g/l glucose, 2mg/l 2,4-D, 100µM acetosyringone and 8.5g/l agar, pH 5.8), Bacterial culture media (YEP Media - Yeast extract - 10g/l, Peptone - 10g/l and Sodium Chloride - 5g/l), Infection Media (MS with 68.5g/l sucrose, 36g/l glucose and 100µM acetosyringone, pH 5.2), Regeneration Media (MS with 30g/l sucrose, 2mg/l BAP and 8.5g/l agar) and Rooting Media (½ MS with 20g/l sucrose, 0.5mg/l IAA and 0.5mg/l NAA)

### Concentration of Agrobacterium during infection

### O.D. of Agrobacterium - between 0.7-1.0

Following the co-cultivation step, or following the resting step, where it is used, the transformed cells are exposed to selective pressure to select for those cells that have received and are expressing polypeptide from the heterologous nucleic acid introduced by *Agrobacterium.* Where the cells are embryos, the embryos are transferred to plates with solid medium that includes both an antibiotic to inhibit growth of the *Agrobacterium* and a selection agent. The agent used to select for transformants will select for preferential growth of explants containing at least one selectable marker insert positioned within the super binary vector and delivered by the *Agrobacterium.*

Generally, any of the media known in the art suitable for the culture of sorghum can be used in the selection step, such as media containing N6 salts or MS salts supplemented with 30 g/l sucrose, 2mg/l 2,4-D and kept in dark for 15 days. During selection, the embryos are cultured until callus formation is observed. Typically, calli grown on selection medium are allowed to grow to a size of about 1.5 to about 2 cm. diameter.

After the calli have reached the appropriate size, the calli are cultured on regeneration medium in the dark for several weeks, generally about 1 to 3 weeks to allow the somatic embryos to mature. Preferred regeneration media include media containing MS media supplemented with 30 g/l sucrose, 2 mg/l BAP and 8.5 g/l agar. The calli are then cultured on rooting medium in a light/dark cycle until shoots and roots develop

Small plantlets are then transferred to tubes containing rooting medium and allowed to grow and develop more roots for approximately another week. The plants are then transplanted to soil mixture in pots in the greenhouse.

### Screening of the dsRNA induced putative transformed lines:

The putative T₀ transformants for both the dsRNA inducing transgenes recovered through hygromycin selection were further screened by PCR analysis to confirm the presence of hygromycin resistant gene. Thus, a PCR analysis was made using the genomic DNA isolated from the transformed individuals along with non-transformed control plant and a set of ++++gene specific primers (5 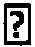 primer: atg aaa aag cct gaa ctc acc gcg acg tct and 3 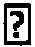 primer: gca tca get cat cga gag cct gcg cga cgg). An amplification of about 547 bp product was observed in all the transformants indicated the presence of hygromycin resistance gene. The amplification was found to be absent in non-transformed *Sorghum* plant. Thus, it could be expected that dsRNA inducing COMT and *CCoAOMT* gene cassettes might have inserted in the genome of the putative T₀ transformants as it was kept linked with the hygromycin resistance gene *(hptII).*

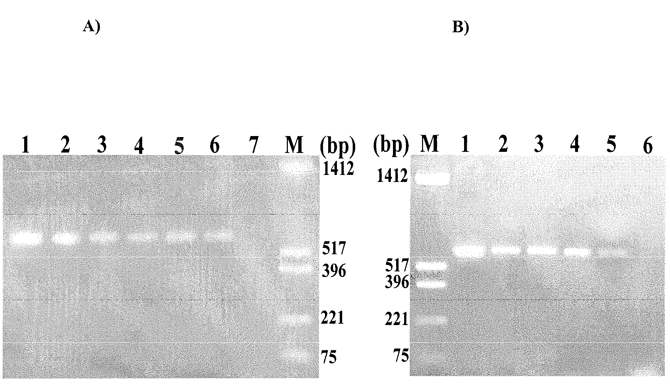

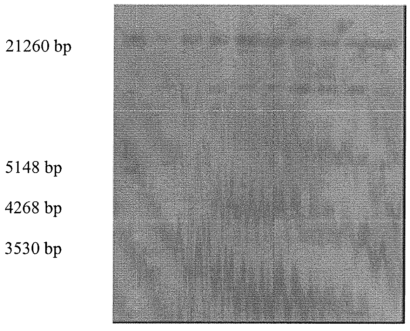

**Southern analysis of transgenic plants:** Sourthern analysis of transgenic plants was done to check the integration pattern as well as copy number of the integrated gene construct. For this, DNA was isolated from control, T0 and T1 plants. 10 mg of DNA of each of the plants was digested with Hindlll and run in a 1% agarose gel. The gel was then transferred onto Nylon membrane (GE healthcare), cross-linked and finally hybridized with COMT and CCoAOMT gene probes respectively (Fig. 6 and 7).

T1 progenies were produced from seeds of self-pollination of T0 plants. Seeds were screened by allowing them to grow in the basal medium containing hygromycin. Eight seeds were found to germinate under hygromycin. The resistant seedlings were then allowed to grow to a mature stage in containment. The plants were analysed for the presence of hygromycin gene by PCR using two gene-specific primers. Finally, Sourthern analysis of the T1 transgenic plants was done to check the integration pattern as well as copy number of the integrated gene construct and single integration was observed in the anti-SEQ ID NO 1 transgenic plants except in T1-1. Moreover, the integration pattern was found similar in all cases. The enzyme activity in T1 plants was found to be almost same in comparison to the T0 plant.

### Analysis of endogenous enzyme activities in transformed lines:

Enzyme activities were analyzed in the tissue of transformed sweet sorghum to assess the alteration of lignin composition. The results have been tabulated in Table 1. Table 2 clearly indicates that down regulation of SEQ ID NO 1 resulted into reduction in lignin content. The result showed that lignin content in stem tissue was reduced to about 14% and 27% in C-plants and T-plants respectively. Table 3 discloses S/G ratio in the transformed plants and control as well. The results showed that S/G ratio was increased in both the plants. The change in S/G ratio was more prominent in T-plant. So, it could be expected that this increase in S/G ratio facilitate the extractability of the cellulosic materials from both the transgenic plant, particularly from T-plant.

**Table 1: Enzyme activity in stem tissue of C and T plants:**

| Lines | COMT activity (pmol/sec/mg of total protein) | CCoAOMT activity (pmol/sec/mg of total protein) |
|---|---|---|
| Control | 4.8 | 14.3 |
| C-plants | 2.3 | 15.3 |
| T-plants | 4.6 | 4.9 |

### Alteration in growth parameters of transgenic lines:

Tthe transgenic lines showed alteration of growth when compared with control. The increment in the plant height was found to be co-related with the increase of internodal length as well as number of internodes. Apart from the increased vegetative growth, an alteration was also observed in seed sizes in transgenic plants compared to control plant. This increase in seed sizes was verified by mean seed weight. The increment was found to be ~ 45% over the control seeds.

### Biochemical analysis of transformed sweet sorghum plants:

### Analysis of lignin content and composition in transgenic plants by chemical method:

Lignin composition with respect to G and S type was determined by derivatization followed by reductive cleavage (DFRC). The monomeric lignin degradation products were identified by GC-FID analysis using acetyl derivative of coniferyl and sinapyl alcohol as standard. The reduction of total lignin content compared to control was ~28% in case of T-plant. But the reduction was to a lesser degree (~14%) in C-plant.

**Table 2: Determination of lignin concentration of transformed Sorghum stem using the Acetyl Bromide Spectrophotometric method:**

| **Stem tissue** | **Acetyl Bromide** lignin content (gm/kg dry cell wall) |
|---|---|
| Control plant | 76.14 |
| C- plant | 65.65 |
| T- plant | 54.84 |

**Table 3: Lignin composition of stem tissue of C and T plants.**

| *Sorghum* plant | S type (%) | G type (%) | S/G ratio |
|---|---|---|---|
| Control plant | 22.29 | 77.71 | 0.29 |
| C-plants | 39.99 | 60.01 | 0.66 |
| T-plants | 63.51 | 36.49 | 1.74 |

### Estimation of cellulose:

A segment from stem (20 mg by fresh weight) of control and transgenic sorghum plants were collected, frozen in liquid nitrogen and crushed to a fine powder. The powder was treated with 3 ml of acetic/nitric reagent (10:1) in boiling water bath for 30 mins, cool and then centrifuged for 15-20 mins. The supernatant was stored to estimate the soluble sugar content. The pellet was washed, treated with 67% sulphuric acid and allowed to stand for 1 hr. The treated sample was then diluted 15 times. 5 ml of anthrone reagent was added to 1 ml of both the treated and untreated sample, mix well, kept in boiling water bath for 10mins, cooled rapidly and measured O.D. at 630nm. The results were expressed as a mean of three samples in each case.

The carbohydrate content of both seed and stem tissue was estimated following the protocols described in Methods. It has been observed that the cellulose content was increased by 48% in C-plants and by 36% in T-plants (Table 4). It is important to note that in spite of higher cellulose content in C-plants (Table 4), the soluble sugar was found to increase more significantly in T-plants (Table 5). Higher content of soluble sugar would help in fermentation process.

**Table 4: Determination of cellulose content in sorghum stem tissue**

| Samples | Cellulose content (mg/g of FW) |
|---|---|
| Control | 86 |
| Anti-COMT line | 127 |
| Anti-CCoAOMT-like line | 117 |

**Table 5: Determination of soluble sugar content in sorghum stem tissue**

| Samples | Soluble sugar content (mg/g of FW) |
|---|---|
| Control | 10.3 |
| Anti-COMT line | 12.1 |
| Anti-CCoAOMT-like line | 29.5 |

### Estimation of starch in seeds:

Seeds were homogenized in hot 80% ethanol to remove sugar and centrifuged. The pellet was washed repeatedly with 80% ethanol and then dried. The pellet was treated with 5 ml of water and 6.5 ml of 52% perchloric acid for 20 mins. The extract was centrifuged and the supernatant was collected. The supernatant was diluted 250 times. 5 ml anthrone reagent was added to 1 ml of the sample, kept at boiling water bath for eight mins and measured at O.D. 630 nm. The results were expressed as a mean of three samples in each case.

The starch content of both seed and stem tissue was estimated following the protocols described in Methods. It has also been observed that starch content in seeds was increased by around 20 and 30% in the C-plants and T-plants respectively (Table 3). It could be suggested that accumulation of starch, apart from other components, may be responsible for increase of seed weight.

**Table 6: Determination starch content in seed**

| Samples content | Average Seed weight | Average Starch |
|---|---|---|
| | (mg) | (mg/seed) |
| Control | 29.4 | 16.6 |
| Anti-COMT line | 38.3 | 20.2 |
| Anti-CCoAOMT-like line | 42.3 | 22.1 |

## Claims

1. A process for producing a transgenic *Sorghum bicolor* plant having increased biomass comprising:
i) transforming or transfecting a *Sorghum bicolor* cell with a double strand mediated gene silencing cassette comprising a nucleic acid represented by SEQ ID NO. 1, wherein said nucleic acid is operably associated with a promoter, and wherein the nucleic acid is in the sense-antisense orientation to generate RNAi in vivo to at least a portion of SEQ ID NO. 1; and
ii) growing a *Sorghum bicolor* plant from said cell.

2. The process according to claim 1, wherein increased biomass is selected from enhanced growth, panicle shape, increased number of seed per panicle, and/or increased size and weight.

3. Use of the plant produced by the process according to claim 1 or 2, comprising the step of harvesting the grain and/or stems of the plant.

4. The use of claim 4 further comprising obtaining alcohol, sugar, syrup or biofuel from said grain and/or stems.

5. Use of the plant produced by the process according to claim 1 or 2 in the production of alcohol, sugar, syrup or biofuel.

## Patentansprüche

1. Verfahren zum Herstellen einer transgenen *Sorghum-bicolor*-Pflanze mit erhöhter Biomasse, umfassend:
(i) Umwandeln oder Transfizieren einer *Sorghum-bicolor-*Zelle mit einer Kassette zur Doppelstrang-vermittelten Genstilllegung, umfassend eine Nukleinsäure, die von SEQ ID NO. 1 dargestellt wird, wobei die Nukleinsäure operabel mit einem Promotor verbunden ist und wobei die Nukleinsäure sich in der Sense-Antisense-Ausrichtung befindet, um RNAi in vivo auf mindestens einem Abschnitt von SEQ ID NO. 1 zu erzeugen; und
(ii) Züchten einer *Sorghum-bicolor-*Pflanze aus der Zelle.

2. Verfahren nach Anspruch 1, wobei die erhöhte Biomasse ausgewählt ist aus verbessertem Wachstum, Rispenform, erhöhter Anzahl an Samen pro Rispe und/oder gesteigerter Größe und gesteigertem Gewicht.

3. Anwendung der mit dem Verfahren nach Anspruch 1 oder 2 hergestellten Pflanze, umfassend den Schritt des Erntens des Getreides und/oder der Stiele der Pflanze.

4. Anwendung nach Anspruch 4, des Weiteren umfassend das Gewinnen von Alkohol, Zucker, Sirup oder Biokraftstoff aus dem Getreide und/oder den Stielen.

5. Anwendung der mit dem Verfahren nach Anspruch 1 oder 2 hergestellten Pflanze bei der Herstellung von Alkohol, Zucker, Sirup oder Biokraftstoff.

## Revendications

1. Procédé de production d'un plant transgénique de sorgho commun (*Sorghum bicolor*) de biomasse accrue, comprenant :
i) la transformation ou la transfection d'une cellule de *Sorghum bicolor* avec une cassette de silençage génique induit à double brin comprenant un acide nucléique représenté par SEQ ID N° : 1, ledit acide nucléique étant fonctionnellement associé à un promoteur et l'acide nucléique se trouvant dans l'orientation sens-antisens afin de générer un ARNi in vivo sur au moins une partie de SEQ. ID N° : 1 ; et
ii) la croissance d'un plant de *Sorghum bicolor* à partir de ladite cellule.

2. Procédé selon la revendication 1, dans lequel la biomasse accrue est choisie parmi une croissance accrue, une forme de panicule, un nombre accru de graines par panicule et/ou une taille et un poids accrus.

3. Utilisation d'un plant produit par le procédé selon la revendication 1 ou 2, comprenant l'étape consistant à récolter les grains et/ou les tiges du plant.

4. Utilisation de la revendication 4 comprenant en outre l'obtention d'alcool, de sucre, de sirop ou de biocarburant à partir desdits grains et/ou tiges.

5. Utilisation du plant produit par le procédé selon la revendication 1 ou 2 dans la production d'alcool, de sucre, de sirop ou de biocarburant.
